(19) **Europäisches Patentamt**
**European Patent Office**
**Office européen des brevets**

(11) **EP 4 597 145 A1**

(12) **EUROPEAN PATENT APPLICATION**

(43) Date of publication:
06.08.2025 Bulletin 2025/32

(51) International Patent Classification (IPC):
*G01R 33/483* (2006.01) *G01R 33/54* (2006.01)

(21) Application number: 25155383.0

(22) Date of filing: 31.01.2025

(52) Cooperative Patent Classification (CPC):
G01R 33/4838; G01R 33/4833; G01R 33/543

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.01.2024 JP 2024013366**

(71) Applicant: **Canon Medical Systems Corporation
Tochigi 324-0036 (JP)**

(72) Inventors:
• **OZAKI, Masanori**
  **Otawara-shi, 324-0036 (JP)**
• **KUSAHARA, Hiroshi**
  **Otawara-shi, 324-0036 (JP)**

(74) Representative: **Marks & Clerk LLP
15 Fetter Lane
London EC4A 1BW (GB)**

(54) **MAGNETIC RESONANCE IMAGING APPARATUS AND MAGNETIC RESONANCE IMAGING METHOD**

(57) A magnetic resonance imaging apparatus (100) according to an embodiment includes an acquisition unit (17b), a measurement unit (17c) and a calculation unit (17d). The acquisition unit (17b) acquires one-dimensional projection data in a phase encoding direction of a subject to be a target of main imaging. The measurement unit (17c) measures a subject thickness in the phase encoding direction using the one-dimensional projection data. The calculation unit (17d) calculates, based on the subject thickness, an excitation thickness and an excitation position of a saturation pulse applied outside an imaging area within the subject in the phase encoding direction when performing the main imaging.

FIG.1

## Description

FIELD

Embodiments described herein relate generally to a magnetic resonance imaging apparatus and a magnetic resonance imaging method.

BACKGROUND

Conventionally, local excitation methods have been widely used as the methods for imaging a small field of view (FOV) in a subject in high detail using a magnetic resonance imaging (MRI) apparatus.

For example, the two-dimensional radio frequency (RF) excitation method, the oblique excitation method, and the outer volume suppression (OVS) method are known as the common local excitation methods. The two-dimensional radio frequency (RF) excitation method is a method of performing local excitation by applying an RF pulse while performing phase encoding. The oblique excitation method is a method of performing local excitation by changing the application angle of an excitation pulse and the application angle of a refocusing pulse. Furthermore, the OVS method is a method of performing local excitation by applying a saturation pulse outside an imaging area in the phase encoding direction to suppress signals outside the imaging area.

Among those, with the OVS method, the excitation thickness and the excitation position of the saturation pulse vary depending on the size of the subject and the position of the imaging area. Therefore, the operator of the MRI apparatus needs to set the excitation thickness and the excitation position of the saturation pulse while visually checking the structure of the subject, thereby complicating the workflow. On the other hand, although it is possible to reduce the complexity of the workflow by setting a large excitation thickness for the saturation pulse, it is desirable to set the excitation thickness as thin as possible since a large excitation thickness deteriorates the excitation profile, resulting in insufficient suppression of signals in the part near the imaging area.

SUMMARY OF INVENTION

A magnetic resonance imaging apparatus, comprising:

an acquisition unit configured to acquire one-dimensional projection data in a phase encoding direction of a subject to be a target of main imaging;
a measurement unit configured to measure a subject thickness in the phase encoding direction using the one-dimensional projection data; and
a calculation unit configured to calculate, based on the subject thickness, an excitation thickness and an excitation position of a saturation pulse applied outside an imaging area within the subject in the phase encoding direction when performing the main imaging.

The acquisition unit may be further configured to acquire the one-dimensional projection data by collecting the one-dimensional projection data in the phase encoding direction by exciting a slice imaging range including a slice of the subject captured in the main imaging.

The measurement unit may be further configured to acquire a signal profile indicating a signal distribution of the subject in the phase encoding direction from the one-dimensional projection data, and measure the subject thickness outside the imaging area in the phase encoding direction based on the signal profile.

The measurement unit may be further configured to specify a position where a signal of the subject becomes a prescribed value in the phase encoding direction based on the signal profile, and measure the subject thickness by measuring a distance between the specified position and an edge position of the imaging area in the phase encoding direction.

The calculation unit may be further configured to calculate the excitation thickness and the excitation position of the saturation pulse based on the subject thickness, a center position of the imaging area, and a size of the imaging area.

The calculation unit may be further configured to calculate a side lobe width of the saturation pulse, and calculate the excitation thickness and the excitation position of the saturation pulse such that the saturation pulse is applied to a space away from the imaging area by at least the size of the side lobe width.

The acquisition unit may be further configured to acquire the one-dimensional projection data by generating the one-dimensional projection data in the phase encoding direction from imaging data for positioning the subject collected before performing the main imaging.

The one-dimensional projection data may be nuclear magnetic resonance data collected by applying only a gradient magnetic field in the phase encoding direction without applying a gradient magnetic field in a frequency encoding direction and a gradient magnetic field in a slice selective excitation direction.

The saturation pulse applied outside the imaging area in the phase encoding direction may be an outer volume suppression (OVS) pulse.

The excitation thickness may be a thickness of the saturation pulse in the phase encoding direction.

The excitation position may be a position of the saturation pulse in the phase encoding direction.

A magnetic resonance imaging method, comprising:

acquiring one-dimensional projection data in a phase encoding direction of a subject to be a target of main imaging;
measuring a subject thickness in the phase encoding direction using the one-dimensional projection data; and
calculating, based on the subject thickness, an excitation thickness and an excitation position of a saturation pulse applied outside an imaging area within the subject in the phase encoding direction when performing the main imaging.

BRIEF DESCRIPTION OF THE DRAWINGS

FIG. 1 is a diagram illustrating a configuration example of an MRI apparatus according to a first embodiment;
FIG. 2 is a diagram illustrating an overview of processing performed by the MRI apparatus according to the first embodiment;
FIG. 3 is a diagram illustrating an example of processing performed by the MRI apparatus according to the first embodiment;
FIG. 4 is a diagram illustrating an example of processing performed by a measurement function according to the first embodiment;
FIG. 5 is a diagram illustrating an example of processing performed by the measurement function according to the first embodiment;
FIG. 6 is a diagram illustrating an example of processing performed by a calculation function according to the first embodiment;
FIG. 7 is a flowchart illustrating a procedure of processing performed by each processing function of the MRI apparatus according to the first embodiment;
FIG. 8 is a diagram illustrating an example of processing performed by an MRI apparatus according to a second embodiment;
FIG. 9 is a diagram illustrating an example of processing performed by the MRI apparatus according to the second embodiment; and
FIG. 10 is a diagram illustrating an example of processing performed by an MRI apparatus according to a third embodiment.

DETAILED DESCRIPTION

An MRI apparatus according to embodiments includes an acquisition unit, a measurement unit, and a calculation unit. The acquisition unit acquires one-dimensional projection data in a phase encoding direction of a subject to be a target of main imaging. The measurement unit measures the subject thickness in the phase encoding direction using the one-dimensional projection data. Based on the subject thickness, the calculation unit calculates the excitation thickness and the excitation position of a saturation pulse that is applied outside an imaging area within the subject in the phase encoding direction when the main imaging is performed.

Hereinafter, embodiments of the MRI apparatus and MRI method according to the present application will be described in detail with reference to the accompanying drawings.

First Embodiment

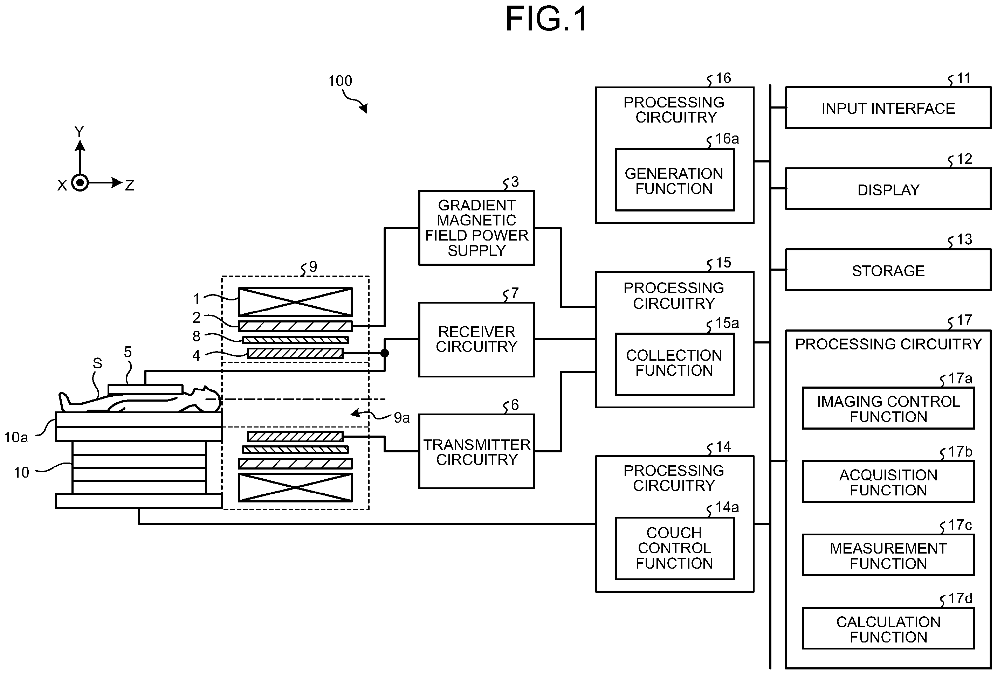

FIG. 1 is a diagram illustrating a configuration example of the MRI apparatus according to a first embodiment.

As illustrated in FIG. 1, an MRI apparatus 100 includes: a static magnetic field magnet 1, a gradient coil 2, a gradient magnetic field power supply 3, a whole-body radio frequency (RF) coil 4, a local RF coil 5, transmitter circuitry 6, receiver circuitry 7, an RF shield 8, a gantry 9, a couch 10, an input interface 11, a display 12, a storage 13, and processing circuitry 14 to processing circuitry 17.

The static magnetic field magnet 1 generates a static magnetic field in an imaging space where a subject S is placed. Specifically, the static magnetic field magnet 1 is formed in a hollow and substantially cylindrical shape (including one whose cross-sectional shape orthogonal to the central axis is elliptical) and generates a static magnetic field in the imaging space formed on the inner circumference side. For example, the static magnetic field magnet 1 is a superconducting magnet, a permanent magnet, or the like. The superconducting magnet herein is configured with a container filled with a coolant such as liquid helium, for example, and a superconducting coil immersed in the container.

The gradient coil 2 is placed inside the static magnetic field magnet 1, and generates a gradient magnetic field in the imaging space where the subject S is placed. Specifically, the gradient coil 2 is formed in a hollow and substantially cylindrical shape (including one whose cross-sectional shape orthogonal to the central axis is elliptical), and has an X coil, a Y coil, and a Z coil respectively corresponding to the mutually orthogonal X, Y, and Z axes. The X coil, the Y coil, and the Z coil generate a gradient magnetic field that changes linearly along each of the axis directions in the imaging space based on the current supplied by the gradient magnetic field power supply 3. Note here that the Z axis is set to be along the magnetic flux of the static magnetic field generated by the static magnetic field magnet 1. The X axis is set to be along the horizontal direction orthogonal to the Z axis, and the Y axis is set to be along the vertical direction orthogonal to the Z axis. Here, the X axis, the Y axis, and the Z axis configure an apparatus coordinate system unique to the MRI apparatus 100.

The gradient magnetic field power supply 3 generates a gradient magnetic field in the imaging space by supplying the current to the gradient coil 2. Specifically, the gradient magnetic field power supply 3 individually supplies the current to the X coil, the Y coil, and the Z coil of the gradient coil 2 to generate, in the imaging space, a gradient magnetic field that linearly changes along each of the frequency encoding direction, the phase encoding direction, and the slice selective excitation direction orthogonal to each other. Here, the axis along the frequency encoding direction, the axis along the phase encoding direction, and the axis along the slice selective excitation direction configure a logical coordinate system for defining a slice area or a volume area to be the target of imaging.

Note here that the gradient magnetic field along each of the frequency encoding direction, the phase encoding direction, and the slice selective excitation direction is superimposed on the static magnetic field generated by the static magnetic field magnet 1, thereby adding spatial positional information to nuclear magnetic resonance (NMR) signals generated from the subject S. Specifically, the gradient magnetic field in the frequency encoding direction changes the frequency of the NMR signal in accordance with the position in the frequency encoding direction, thereby adding positional information in the frequency encoding direction to the NMR signal. Furthermore, the gradient magnetic field in the phase encoding direction changes the phase of the NMR signal in accordance with the position in the phase encoding direction, thereby adding positional information in the phase encoding direction to the NMR signal. When two-dimensional MR images (slice images) are to be captured, the gradient magnetic field in the slice selective excitation direction changes the frequency of the NMR signal in accordance with the position in the slice selective excitation direction, thereby determining the position, thickness, and number of slices to be imaged. Furthermore, when three-dimensional MR images (volume images) are to be captured, the gradient magnetic field in the slice selective excitation direction changes the phase of the NMR signal in accordance with the position in the slice selective excitation direction, thereby adding the positional information in the slice selective excitation direction to the NMR signal.

The whole-body RF coil 4 is placed on the inner circumference side of the gradient coil 2, applies the RF pulse (excitation pulse or the like) to the subject S placed in the imaging space, and receives the NMR signal (echo signal or the like) generated from the subject S by the effect of the RF pulse. Specifically, the whole-body RF coil 4 is formed in a hollow and substantially cylindrical shape (including one whose cross-sectional shape orthogonal to the central axis is elliptical) and applies the RF pulse to the subject S placed in the imaging space on the inner circumference side thereof based on the RF pulse signal supplied from the transmitter circuitry 6. Then, the whole-body RF coil 4 receives the NMR signal generated from the subject S by the effect of the RF pulse, and outputs the received NMR signal to the receiver circuitry 7. For example, the whole-body RF coil 4 is a birdcage coil or a transverse electromagnetic (TEM) coil.

The local RF coil 5 is placed near the subject S during imaging, and receives the NMR signal generated from the subject S. Specifically, the local RF coil 5 is prepared for each site of the subject S, is placed near the site of imaging target when imaging the subject S, receives the NMR signal generated from the subject S by the effect of the RF pulse applied by the whole-body RF coil 4, and outputs the received NMR signal to the receiver circuitry 7. For example, the local RF coil 5 is a surface coil or a phased array coil configured with a plurality of surface coils combined as coil elements. Note that the local RF coil 5 may further have a transmission function to apply the RF pulse to the subject.

The transmitter circuitry 6 outputs the RF pulse signal corresponding to the resonance frequency (Larmor frequency) unique to the target nucleus placed in the static magnetic field to the whole-body RF coil 4 or the local RF coil 5. Specifically, the transmitter circuitry 6 includes a pulse generator, an RF generator, a modulator, and an amplifier. The pulse generator generates the waveforms of RF pulse signals. The RF generator generates RF signals at the resonance frequency. The modulator generates the RF pulse signal by modulating the amplitude of the RF signal generated by the RF generator with the waveform generated by the pulse generator. The amplifier amplifies and outputs the RF pulse signal generated by the modulator to the whole-body RF coil 4 or the local RF coil 5.

The receiver circuitry 7 generates NMR data based on the NMR signals output from the whole-body RF coil 4 or the local RF coil 5, and outputs the generated NMR data to the processing circuitry 15. Specifically, the receiver circuitry 7 includes a selector, a preamplifier, a phase detector, and an analog/digital (A/D) converter. The selector selectively inputs the NMR signals output from the whole-body RF coil 4 or the local RF coil 5. The preamplifier amplifies the NMR signal output from the selector. The phase detector detects the phase of the NMR signal output from the preamplifier. The A/D converter generates NMR data by converting analog signals output from the phase detector into digital signals, and outputs the generated NMR data to the processing circuitry 15. Note here that all of various kinds of processing described to be performed by the receiver circuitry 7 are not necessarily need to be performed by the receiver circuitry 7, and some processing (for example, processing by the A/D converter and the like) may be performed by the whole-body RF coil 4 or the local RF coil 5.

The RF shield 8 is placed between the gradient coil 2 and the whole-body RF coil 4 to shield the gradient coil 2 from the RF pulses generated by the whole-body RF coil 4. Specifically, the RF shield 8 is formed in a hollow and substantially cylindrical shape (including one whose cross-sectional shape orthogonal to the central axis of the cylinder is elliptical), and is placed in the space on the inner circumference side of the gradient coil 2 to cover the outer circumference face of the whole-body RF coil 4.

The gantry 9 has a hollow bore 9a formed in a substantially cylindrical shape (including one whose cross-sectional shape orthogonal to the central axis is elliptical), and houses the static magnetic field magnet 1, the gradient coil 2, the whole-body RF coil 4, and the RF shield 8. Specifically, the gantry 9 houses each of those with the whole-body RF coil 4 being placed on the outer circumference side of the bore 9a, the RF shield 8 being placed on the outer circumference side of the whole-body RF coil 4, the gradient coil 2 being placed on the outer circumference side of the RF shield 8, and the static magnetic field magnet 1 being placed on the outer circumference side of the gradient coil 2. Note here that the space within the bore 9a of the gantry 9 is the imaging space in which the subject S is placed during imaging.

A couch 10 has a couchtop 10a on which the subject S is placed, and when imaging of the subject S is to be performed, the couchtop 10a on which the subject S is placed is moved to the imaging space. For example, the couch 10 is installed such that the longitudinal direction of the couchtop 10a is parallel to the central axis of the static magnetic field magnet 1.

Here, while an example of a case is described in which the MRI apparatus 100 has a so-called tunnel-type structure in which the static magnetic field magnet 1, the gradient coil 2, and the whole-body RF coil 4 are each formed in a substantially cylindrical shape, the embodiment is not limited thereto. For example, the MRI apparatus 100 may have a so-called open-type structure in which a pair of static magnetic field magnets, a pair of gradient coils, and a pair of RF coils are arranged to face each other by sandwiching the imaging space where the subject S is placed. In such an open-type structure, the space sandwiched between a pair of static magnetic field magnets, a pair of gradient coils, and a pair of RF coils corresponds to the bore in the tunnel-type structure.

The input interface 11 accepts input operations of various kinds of instructions and various kinds of information from an operator. Specifically, the input interface 11 is connected to the processing circuitry 17, which converts input operations received from the operator into electrical signals and outputs those to the processing circuitry 17. For example, the input interface 11 is implemented by a trackball, a switch button, a mouse, a keyboard, a touch pad for performing input operations by touching an operation face, a touch screen where a display screen and a touch pad are integrated, non-contact input circuitry using an optical sensor, audio input circuitry, and the like for performing setting of the imaging condition and the region of interest (ROI), and the like. Note that the input interface 11 herein is not limited only to those with physical operation components such as a mouse, keyboard, and the like. For example, electrical signal processing circuitry that receives electrical signals corresponding to input operations from an external input device provided separately from the apparatus and outputs the electrical signals to control circuitry is also an example of the input interface 11.

The display 12 displays various kinds of information. Specifically, the display 12 is connected to processing circuitry 17, and converts and outputs data of various kinds of information transmitted from the processing circuitry 17 into electrical signals for display. For example, the display 12 can be implemented by a liquid crystal monitor, a cathode ray tube (CRT) monitor, a touch panel, or the like.

The storage 13 stores various kinds of data. Specifically, the storage 13 is connected to each piece of the processing circuitry 14 to 17, and stores various kinds of data input and output by each piece of the processing circuitry. For example, the storage 13 is implemented by a semiconductor memory element such as a random access memory (RAM) or a flash memory, or by a hard disk (hard disk), an optical disk, or the like.

The processing circuitry 14 has a couch control function 14a. The couch control function 14a controls the motion of the couch 10 by outputting control electrical signals to the couch 10. For example, the couch control function 14a receives an instruction from the operator via the input interface 11 to move the couchtop 10a in the longitudinal direction, the up-and-down direction, or the left-and-right direction, and operates a moving mechanism of the couchtop 10a of the couch 10 so as to move the couchtop 10a according to the received instruction.

The processing circuitry 15 has a collection function 15a. The collection function 15a collects NMR data from the subject S by executing various kinds of pulse sequences. Specifically, the collection function 15a executes various kinds of pulse sequences by driving the gradient magnetic field power supply 3, the transmitter circuitry 6, and the receiver circuitry 7 according to sequence execution data output from the processing circuitry 17. Note here that the sequence execution data is data that represents the pulse sequence, and it is information that defines the timing and intensity of the current supplied by the gradient magnetic field power supply 3 to the gradient coil 2, the timing of the RF pulse signal and intensity of the RF pulse signal supplied by the transmitter circuitry 6 to the whole-body RF coil 4, the timing at which the receiver circuitry 7 samples the NMR signal (sampling), and the like. The collection function 15a receives the NMR data output from the receiver circuitry 7 as a result of executing the pulse sequence, and stores it in the storage 13. At this time, the NMR data stored in the storage 13 is stored as k-space data representing two-dimensional or three-dimensional k-space with the positional information added along each of the frequency encoding direction, phase encoding direction, and slice selective excitation direction by the respective gradient magnetic fields described above.

The processing circuitry 16 has a generation function 16a. The generation function 16a generates MR images from the NMR data collected by the collection function 15a of the processing circuitry 15. Specifically, the generation function 16a reads the NMR data collected by the collection function 15a of the processing circuitry 15 from the storage 13 under the control of the processing circuitry 17, and applies reconstruction processing such as Fourier transform or the like to the read NMR data to generate two-dimensional or three-dimensional MR images. Then, the generation function 16a stores the generated MR images in the storage 13.

The processing circuitry 17 has an imaging control function 17a, an acquisition function 17b, a measurement function 17c, and a calculation function 17d. The imaging control function 17a performs overall control of the MRI apparatus 100 by controlling each structural component of the MRI apparatus 100. Specifically, the imaging control function 17a displays a graphical user interface (GUI) on the display 12 for receiving input operations of various kinds of instructions and various kinds of information from the operator, and controls each structural component of the MRI apparatus 100 according to the input operations received via the input interface 11. For example, the imaging control function 17a receives input of imaging condition from the operator, and sets the pulse sequence for collecting the NMR data of the subject S based on the inputted imaging condition. The imaging control function 17a then generates sequence execution data representing the set pulse sequences, and outputs it to the processing circuitry 15 to cause the collection function 15a of the processing circuitry 15 to execute various kinds of pulse sequences. Furthermore, for example, the imaging control function 17a controls the generation function 16a of the processing circuitry 16 to reconstruct MR images from the k-space data collected by the processing circuitry 15. Moreover, for example, the imaging control function 17a reads out the MR images stored in the storage 13 in response to a request from the operator, and displays the read MR images on the display 12. The acquisition function 17b, the measurement function 17c, and the calculation function 17d will be described later.

Note here that each piece of the processing circuitry 14 to 17 is implemented by, for example, a processor. In this case, the processing functions of the processing circuitry are stored in the storage 13 in the form of a computer program that can be executed by a computer, for example. Then, the processing circuitry reads out and executes the computer programs from the storage 13 to implement the processing functions corresponding to the computer programs. In other words, the processing circuitry comes to have respective processing functions illustrated in FIG. 1 by reading out the respective programs.

An example of the configuration of the MRI apparatus 100 according to the present embodiment is described heretofore. Under such a configuration, the MRI apparatus 100 according to the present embodiment is provided with the function of imaging the subject using the OVS method that is one of the local excitation methods capable of imaging a small imaging area of the subject in high detail. Note here that the OVS method is a method of performing local excitation by applying a saturation pulse outside the imaging area in the phase encoding direction to suppress signals outside the imaging area.

Generally, with the OVS method, the excitation thickness and the excitation position of the saturation pulse vary depending on the size of the subject and the position of the imaging area. Therefore, the operator of the MRI apparatus needs to set the excitation thickness and the excitation position of the saturation pulse while visually checking the structure of the subject, thereby complicating the workflow. On the other hand, although it is possible to reduce the complexity of the workflow by setting a large excitation thickness for the saturation pulse, it is desirable to set the excitation thickness as thin as possible since a large excitation thickness deteriorates the excitation profile, resulting in insufficient suppression of signals in the part near the imaging area.

Therefore, the MRI apparatus 100 according to the present embodiment is configured to be able to easily and appropriately set the excitation thickness and the excitation position of the saturation pulse that is applied outside the imaging area within the subject in the phase encoding direction.

Hereinafter, the configuration of such MRI apparatus 100 will be described in detail. In the following, a saturation pulse applied outside the imaging area within the subject in the phase encoding direction when performing imaging using the OVS method is referred to as an OVS pulse.

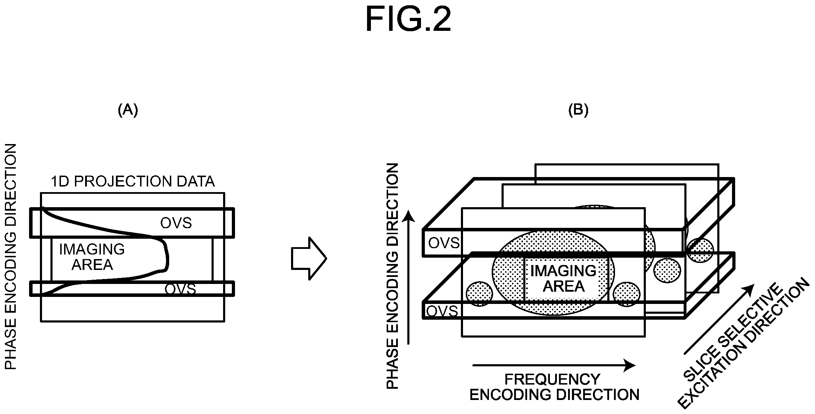

FIG. 2 is a diagram illustrating an overview of processing performed by the MRI apparatus 100 according to the first embodiment.

As illustrated in FIG. 2, when performing the main imaging using the OVS method, the MRI apparatus 100 measures the subject thickness in the phase encoding direction using one-dimensional (1-dimension: 1D) projection data in the phase encoding direction of the subject as the target of the main imaging (see (A) of FIG. 2).

Note here that 1D projection data in the phase encoding direction is data of the subject that is projected in the frequency encoding direction and the slice selective excitation direction as the projection directions with respect to one axis along the phase encoding direction, which includes a signal profile indicating signal distributions of the subject in the phase encoding direction.

Then, the MRI apparatus 100 automatically calculates the excitation thickness and the excitation position of the OVS pulse based on the measured subject thickness (see (B) of FIG. 2). Note here that the excitation thickness is the thickness of the OVS pulse in the phase encoding direction. The excitation position is the position of the OVS pulse in the phase encoding direction.

Specifically, the acquisition function 17b of the processing circuitry 17 acquires 1D projection data in the phase encoding direction of the subject to be the target of main imaging. Furthermore, the measurement function 17c of the processing circuitry 17 measures the subject thickness in the phase encoding direction using the 1D projection data acquired by the acquisition function 17b. The calculation function 17d of the processing circuitry 17 calculates the excitation thickness and the excitation position of the OVS pulse based on the subject thickness measured by the measurement function 17c. Note here that the acquisition function 17b is an example of an acquisition unit. The measurement function 17c is an example of a measurement unit. Furthermore, the calculation function 17d is an example of a calculation unit.

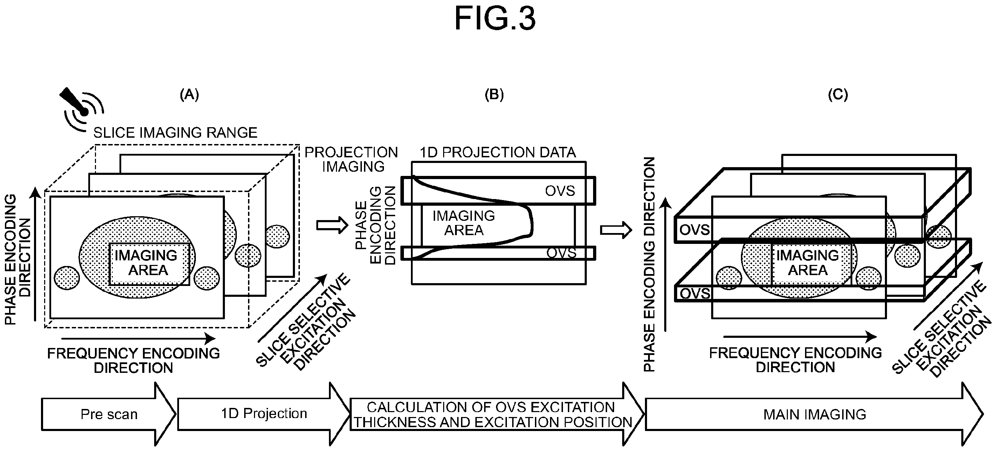

FIG. 3 is a diagram illustrating an example of processing performed by the MRI apparatus 100 according to the first embodiment.

For example, as illustrated in FIG. 3, the acquisition function 17b acquires the 1D projection data (see (A) of FIG. 3) by collecting, in the pre-scan performed before the main imaging, 1D projection data in the phase encoding direction by exciting the slice imaging range including the slice of the subject to be imaged in the main imaging.

For example, the acquisition function 17b controls the collection function 15a of the processing circuitry 15 to collect the 1D projection data in the phase encoding direction by performing slab excitation in the slice imaging range at the position of the center coordinates of the imaging area so as to acquire the 1D projection data in the phase encoding direction. When a plurality of slices of the subject are to be imaged in main imaging, the slice imaging range is set to include those slices. When there is only one slice to be imaged in the main imaging, there may be only one slice included in the slice imaging range.

Thereafter, before the main imaging is performed, the measurement function 17c measures the subject thickness in the phase encoding direction by using the 1D projection data acquired by the acquisition function 17b (see (B) of FIG. 3), and the calculation function 17d calculates the excitation thickness and the excitation position of the OVS pulse based on the subject thickness measured by the measurement function 17c (see (C) of FIG. 3).

For example, the measurement function 17c acquires a signal profile indicating the signal distribution of the subject in the phase encoding direction from the 1D projection data, and measures the subject thickness outside the imaging area in the phase encoding direction based on the signal profile.

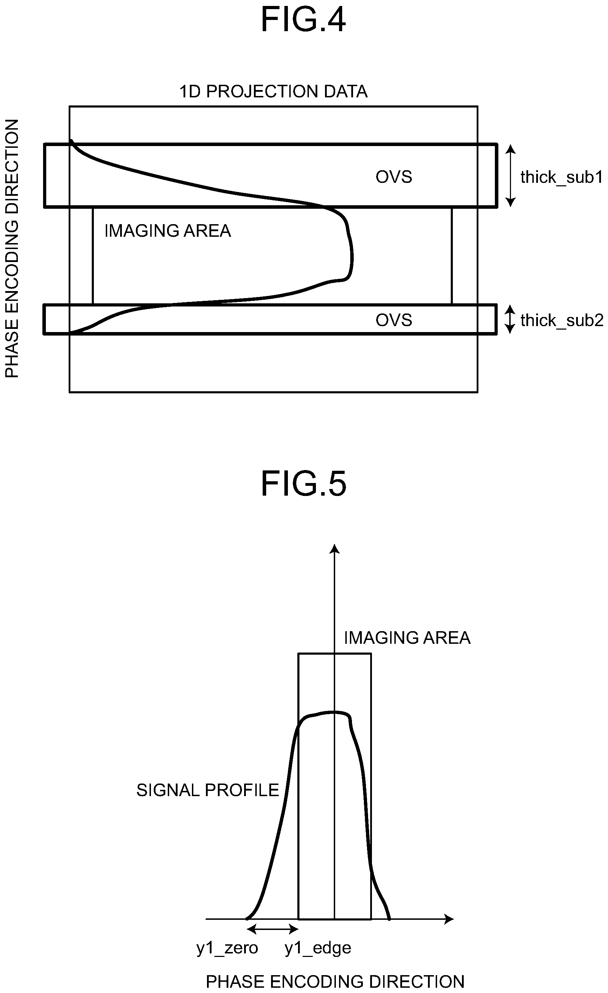

FIGS. 4 and 5 are diagrams illustrating examples of processing performed by the measurement function 17c according to the first embodiment.

For example, as illustrated in FIG. 4, the measurement function 17c measures a subject thickness thick_sub1 on the outer side of one side of the imaging area (upper side in FIG. 4) in the phase encoding direction and a subject thickness thick_sub2 on the outer side of the other side of the imaging area (lower side in FIG. 4) based on the signal profile acquired from the 1D projection data.

In this case, for example, the measurement function 17c specifies the position where the signal of the subject becomes a prescribed value in the phase encoding direction based on the signal profile acquired from the 1D projection data, and measures the distance between the specified position and the edge position of the imaging area in the phase encoding direction so as to measure the subject thickness outside the imaging area in the phase encoding direction.

For example, the measurement function 17c acquires the signal profile from the 1D projection data after removing the background noise contained in the 1D projection data, and specifies the position where the signal of the subject becomes zero in the phase encoding direction based on the signal profile. Then, the measurement function 17c measures the subject thickness outside the imaging area in the phase encoding direction by measuring the distance between the specified position where the signal of the subject is zero and the edge position of the imaging area in the phase encoding direction.

For example, as illustrated in FIG. 5, the measurement function 17c specifies, based on the signal profile acquired from the 1D projection data, a coordinate y1_zero at the position where the signal of the subject is zero and a coordinate y1_edge at the position of the edge of the imaging area for one side of the phase encoding direction from the center coordinates of the imaging area. Then, the measurement function 17c measures the subject thickness thick_sub1 outside the imaging area on one side of the phase encoding direction by measuring a distance abs(y1_zero-y1_edge) between the specified coordinate y1_zero and coordinate y1_edge. Furthermore, the measurement function 17c also performs the same processing for the other side of the phase encoding direction from the center coordinates of the imaging area so as to measure the subject thickness thick_sub2 outside the imaging area on the other side of the phase encoding direction.

Although it is described herein that the measurement function 17c specifies the position where the signal of the subject is zero in the phase encoding direction after removing the background noise contained in the 1D projection data, the embodiment is not limited thereto. For example, the measurement function 17c may use a threshold set to a magnitude that can remove the background noise, and specifies the position where the signal of the subject becomes the threshold in the phase encoding direction. In this case, the measurement function 17c does not need to remove the background noise in the 1D projection data.

Furthermore, for example, the calculation function 17d calculates the excitation thickness and the excitation position of the OVS pulse based on the subject thickness measured by the measurement function 17c, the center position of the imaging area, and the size of the imaging area.

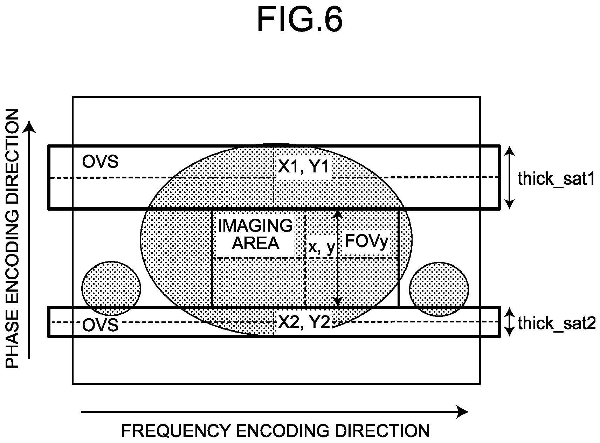

FIG. 6 is a diagram illustrating an example of processing performed by the calculation function 17d according to the first embodiment.

For example, as illustrated in FIG. 6, the calculation function 17d calculates an excitation thickness thick_sut1 of a first OVS pulse applied on the outer side of one side of the imaging area (upper side in FIG. 6) in the phase encoding direction and an excitation thickness thick_sut2 of a second OVS pulse applied on the outer side of the other side of the imaging area (lower side in FIG. 6).

For example, the calculation function 17d calculates the excitation thickness thick_sut1 of the first OVS pulse to become the same size as the subject thickness thick_sub1 on the outer side of one side of the imaging area measured by the measurement function 17c. The calculation function 17d also calculates the excitation thickness thick_sut2 of the second OVS pulse to become the same size as the subject thickness thick_sub2 on the outer side of the other side of the imaging area measured by the measurement function 17c.

Furthermore, for example, assuming that the coordinate in the frequency encoding direction is expressed as x or X, the coordinate in the phase encoding direction is expressed as y or Y, the size of the imaging area in the phase encoding direction is FOVy, the center coordinates of the imaging area are (x, y), the excitation center coordinates of the first OVS pulse are (X1, Y1), the excitation center coordinates of the second OVS pulse are (X2, Y2), the calculation function 17d calculates the coordinate Y1 of the excitation center coordinates of the first OVS pulse in the phase encoding direction and the coordinate Y2 of the excitation center coordinates of the second OVS pulse in the phase encoding direction using the following equations.

$$Y1 = y + FOVy/2 + thick\_sat1/2$$

$$Y2 = y - FOVy/2 - thick\_sat1/2$$

Furthermore, for example, the calculation function 17d receives an excitation width OVSx of the OVS pulse in the frequency encoding direction from the operator, and calculates the coordinate X1 of the first OVS pulse in the frequency encoding direction and the coordinate X2 of the second OVS pulse in the frequency encoding direction using the equations: X1 = OVSx/2 and X2 = OVSx/2.

Then, after the excitation thickness and the excitation position of the OVS pulse are calculated by the calculation function 17d, the imaging control function 17a of the processing circuitry 17 applies the OVS pulse based on the calculated excitation thickness and the excitation position to perform the main imaging of the subject using the OVS method.

Each of the processing functions of the processing circuitry 17 is described heretofore. The processing circuitry 17, as described above, is implemented by a processor, for example. In this case, each of the processing functions of the processing circuitry 17 is stored in the storage 13 in the form of a computer program that can be executed by a computer, for example. Furthermore, the processing circuitry 17 reads out and executes the computer program from the storage 13 to implement the processing functions corresponding to the computer program.

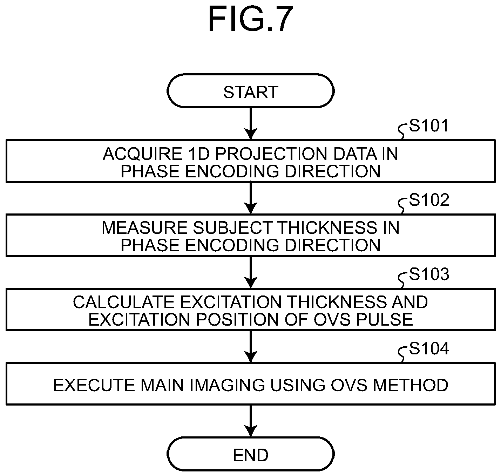

FIG. 7 is a flowchart illustrating a procedure of processing performed by each of the processing functions of the MRI apparatus 100 according to the first embodiment.

For example, as illustrated in FIG. 7, in the present embodiment, first, the acquisition function 17b acquires 1D projection data in the phase encoding direction of the subject to be the target of main imaging (step S101). The processing at step S101 is achieved, for example, by reading out and executing, by the processing circuitry 17, a prescribed computer program corresponding to the acquisition function 17b from the storage 13.

Then, the measurement function 17c measures the subject thickness in the phase encoding direction using the 1D projection data acquired by the acquisition function 17b (step S102). The processing at step S102 is achieved, for example, by reading out and executing, by the processing circuitry 17, a prescribed computer program corresponding to the measurement function 17c from the storage 13.

Thereafter, the calculation function 17d calculates the excitation thickness and the excitation position of the OVS pulse based on the subject thickness measured by the measurement function 17c (step S103). The processing at step S103 is achieved, for example, by reading out and executing, by the processing circuitry 17, a prescribed computer program corresponding to the calculation function 17d from the storage 13.

Then, the imaging control function 17a executes main imaging of the subject using the OVS method by applying the OVS pulse based on the excitation thickness and the excitation position calculated by the calculation function 17d (step S104). The processing at step S104 is achieved, for example, by reading out and executing, by the processing circuitry 17, a prescribed computer program corresponding to the imaging control function 17a from the storage 13.

As described above, in the first embodiment, the acquisition function 17b acquires 1D projection data in the phase encoding direction of the subject to be the target of main imaging. Furthermore, the measurement function 17c measures the subject thickness in the phase encoding direction using the 1D projection data acquired by the acquisition function 17b. Also, the calculation function 17d calculates the excitation thickness and the excitation position of the OVS pulse based on the subject thickness measured by the measurement function 17c.

Such a configuration eliminates the need for the operator of the MRI apparatus to set the excitation thickness and the excitation position of the OVS pulse, which results in improving the workflow. In addition, regardless of the size of the subject or the position of the imaging area, the OVS pulse can be applied with the minimum excitation thickness, thereby making it possible to perform local excitation appropriately. Therefore, according to the first embodiment, it is possible to easily and appropriately set the excitation thickness and the excitation position of the OVS pulse.

While the first embodiment is described heretofore, the first embodiment described above can also be implemented by changing some of the processing functions of the processing circuitry 17 of the MRI apparatus 100 as appropriate. Thus, some modification examples regarding the first embodiment will be described below as other embodiments. In the following embodiments, the points different from the first embodiment will be focused, and detailed explanations of duplicated content will be omitted.

Second Embodiment

For example, in the first embodiment described above, the calculation function 17d calculates the excitation thickness and the excitation position of the OVS pulse based on the subject thickness measured by the measurement function 17c, the center position of the imaging area, and the size of the imaging area. However, in general, it is known that the RF profile of the OVS pulse is not rectangular but has a side lobe, and aliasing occurs at the side lobe part.

Thus, for example, the calculation function 17d may calculate the excitation thickness and the excitation position of the OVS pulse by considering the side lobe of the OVS pulse to suppress aliasing caused by the side lobe of the OVS pulse. Hereinafter, such a case will be described as a second embodiment.

For example, the calculation function 17d calculates the side lobe width of the OVS pulse and calculates the excitation thickness and the excitation position of the OVS pulse such that the OVS pulse is applied to a space away from the imaging area by at least the size of the side lobe width.

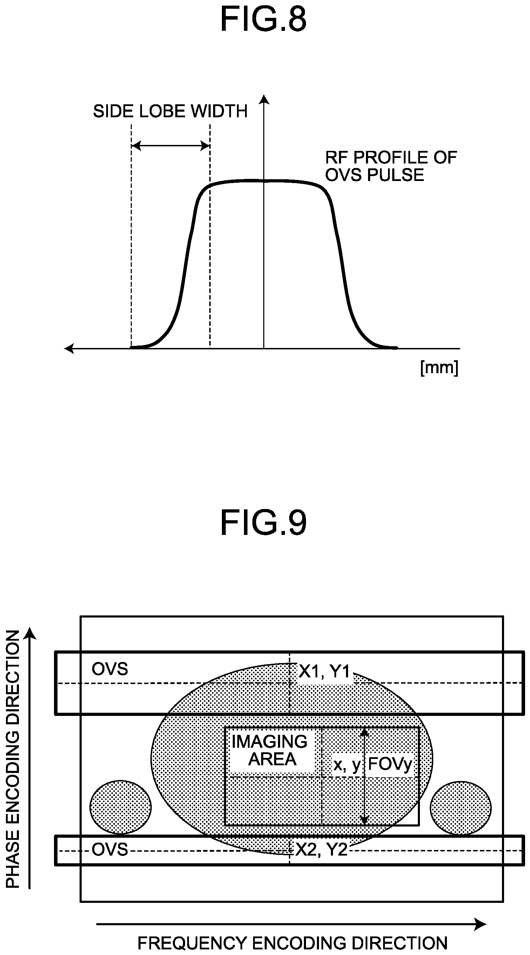

FIGS. 8 and 9 are diagrams illustrating an example of processing performed by the MRI apparatus 100 according to the second embodiment.

For example, as illustrated in FIG. 8, after calculating the excitation thickness thick_sut1 of the first OVS pulse applied on the outer side of one side of the imaging area in the phase encoding direction and the excitation thickness thick_sut2 of the second OVS pulse applied on the outer side of the other side of the imaging area by the same method as that of the first embodiment, the calculation function 17d calculates the RF profile of the OVS pulse for each of the OVS pulses, and calculates the side lobe width of the RF profile.

Furthermore, as illustrated in FIG. 9, for example, assuming that the coordinate in the frequency encoding direction is expressed as x or X, the coordinate in the phase encoding direction is expressed as y or Y, the size of the imaging area in the phase encoding direction is FOVy, the center coordinates of the imaging area are (x, y), the excitation center coordinates of the first OVS pulse are (X1, Y1), the excitation center coordinates of the second OVS pulse are (X2, Y2), the calculation function 17d calculates the coordinate Y1 of the excitation center coordinates of the first OVS pulse in the phase encoding direction and the coordinate Y2 of the excitation center coordinates of the second OVS pulse in the phase encoding direction using the following equations.

Y1 = y + FOVy/2 + thick_sat1/2 + side lobe width of first OVS pulse Y2 = y - FOVy/2 - thick_sat1/2 - side lobe width of second OVS pulse

Thereby, each of the first OVS pulse and the second OVS pulse is applied to a space away from the imaging area by the size of the side lobe width. Note that the space between the OVS pulse and the imaging area does not necessarily need to match the side lobe width of the OVS pulse. For example, it may also be a space in which a predetermined width is added to the side lobe width of the OVS pulse such that aliasing is more securely suppressed.

In the second embodiment described above, the acquisition function 17b calculates the side lobe width of the OVS pulse, and calculates the excitation thickness and the excitation position of the OVS pulse such that the OVS pulse is applied to a space away from the imaging area by at least the size of the side lobe width.

Therefore, according to the second embodiment, aliasing caused by the side lobe of the OVS pulse can be suppressed. In addition, it is possible to prevent redoing of the imaging in a case of aliasing, thereby making it possible to suppress prolongation of the time required for imaging.

Third Embodiment

Furthermore, in the first embodiment described above, for example, the acquisition function 17b acquires the 1D projection data by collecting, in the pre-scan performed before the main imaging, 1D projection data in the phase encoding direction by exciting the slice imaging range including the slice of the subject to be imaged in the main imaging. However, the method of collecting the 1D projection data is not limited thereto.

For example, the acquisition function 17b may generate 1D projection data in the phase encoding direction from imaging data that is generally collected in the pre-scan so as to acquire 1D projection data used to calculate the excitation thickness and the excitation position of the OVS pulse. Hereinafter, such a case will be described as a third embodiment.

For example, the acquisition function 17b acquires 1D projection data by generating 1D projection data in the phase encoding direction from imaging data for positioning the subject collected before the main imaging.

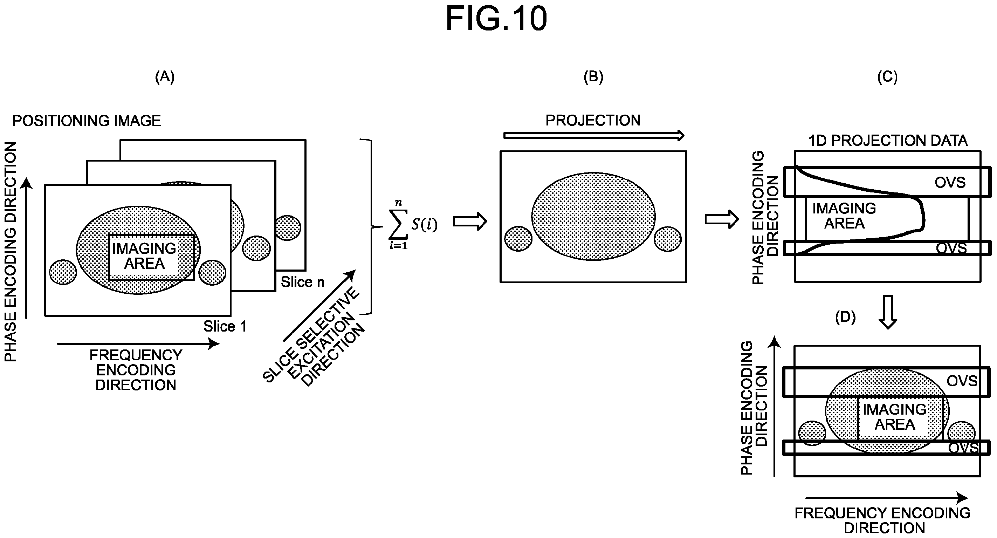

FIG. 10 is a diagram illustrating an example of processing performed by the MRI apparatus 100 according to the third embodiment.

For example, as illustrated in FIG. 10, the acquisition function 17b acquires the positioning images of a plurality of slices (Slice 1 to Slice n) of the subject collected in the pre-scan performed before the main imaging from the storage 13 (see (A) of FIG. 10).

The positioning image (also referred to as locator image) is an image of the subject used to determine the position of the imaging area in the main imaging, such as multi-slice data, volume data, or the like of the subject.

Then, the acquisition function 17b virtually generates 1D projection data in the phase encoding direction by integrating signals of a plurality of acquired positioning images in the slice selective excitation direction and then performing projection with the frequency encoding direction as the projection direction (see (B) of FIG. 10).

Thereafter, as in the first embodiment, the measurement function 17c measures the subject thickness in the phase encoding direction by using the 1D projection data acquired by the acquisition function 17b (see (C) of FIG. 10), and the calculation function 17d calculates the excitation thickness and the excitation position of the OVS pulse based on the subject thickness measured by the measurement function 17c (see (D) of FIG. 10).

Although it is described herein that the acquisition function 17b generates 1D projection data in the phase encoding direction from the positioning images of the subject, it is also possible to use other kinds of imaging data collected in general in pre-scan instead of the positioning images. For example, the acquisition function 17b may generate 1D projection data in the phase encoding direction from sensitivity map data for luminance correction or for parallel imaging collected in pre-scan.

As described above, in the third embodiment, the acquisition function 17b acquires 1D projection data used to calculate the excitation thickness and the excitation position of the OVS pulse by generating 1D projection data in the phase encoding direction from the imaging data such as the positioning image or sensitive map collected generally in the pre-scan before the main imaging is performed.

Therefore, according to the third embodiment, there is no need to add, to the pre-scan, additional data collection for calculating the excitation thickness and the excitation position of the OVS pulse, thereby making it possible to suppress prolongation of the time required for imaging.

The first to third embodiments are described heretofore. However, for example, in the first and second embodiments, one-dimensional projection data may be MR data that is collected by applying only the gradient magnetic field in the phase encoding direction, without applying the gradient magnetic field in the frequency encoding direction and the gradient magnetic field in the slice selective excitation direction. This makes it possible to shorten the collection time of one-dimensional projection data and to easily and appropriately set the excitation thickness and the excitation position of the OVS pulse without prolonging the overall imaging time.

Furthermore, while the first through third embodiments described above illustrate the case of calculating the excitation thickness and the excitation position of the OVS pulse used when imaging a three-dimensional imaging area, the methods described in each of the embodiments are also applicable to the case of calculating the excitation thickness and the excitation position of a two-dimensional saturation pulse that is applied outside the imaging area in the phase encoding direction when imaging a two-dimensional imaging area.

Another Embodiment

While each piece of the processing circuitry 14 to 17 in the above embodiments is described to be implemented by a single processor, the embodiments are not limited thereto. For example, the processing circuitry may be configured with a combination of a plurality of independent processors to implement each of the processing functions by executing the computer programs with each of the processors. Furthermore, the processing functions of the processing circuitry may be distributed or integrated into a single piece of or a plurality of pieces of processing circuitry as appropriate. While an example in which a single storage 13 stores computer programs corresponding to each of the processing functions is described above, the embodiments are not limited thereto. For example, a plurality of storages may be arranged in a distributed manner for each piece of the processing circuitry, and each piece of the processing circuitry may read out the corresponding computer program from the individual storage.

Furthermore, while the embodiments described above illustrate examples of the case where the acquisition unit, the measurement unit, and the calculation unit in the present specification are implemented by the acquisition function, the measurement function, and the display control function of the processing circuitry, respectively, embodiments are not limited thereto. For example, other than being implemented by the acquisition function, the measurement function, and the calculation function described in the embodiments above, the acquisition unit, the measurement unit, and the calculation unit in the present specification may be implemented by hardware only, software only, or a mixture of hardware and software.

While an example in which a "processor" reads out and executes a computer program corresponding to each of the processing functions from a storage is described above, the embodiments are not limited thereto. The term "processor" means, for example, a circuit such as a central processing unit (CPU), a graphics processing unit (GPU), an application specific integrated circuit (ASIC), a programmable logic device (for example, a simple programmable logic device (SPLD), a complex programmable logic device (CPLD), and a field programmable gate array (FPGA)). When the processor is a CPU, for example, the processor reads out and executes the computer program stored in the storage to implement each of the processing functions. In the meantime, when the processor is ASIC, instead of saving the computer program in the storage, the processing functions may be directly incorporated as a logic circuit in the circuitry of the processor. Note that each of the processors of the present embodiment is not limited to being configured as a single circuit for each processor, and may also be configured as a single processor by combining a plurality of independent circuits to implement the processing functions. Furthermore, it is also possible to integrate a plurality of structural components in FIG. 1 into a single processor to implement the processing functions.

Note here that the computer program to be executed by the processor is provided by being installed in advance in a read-only memory (ROM), a storage, or the like. The computer program may be provided in a file of format that can be installed on such devices or in an executable format by being recorded on a computer readable storage medium such as a compact disc (CD)-ROM, a flexible disk (FD), a CD-recordable (CD-R), a digital versatile disc (DVD), or the like. Furthermore, the computer program may also be stored on a computer connected to a network such as the Internet, and provided or distributed by being downloaded via the network. For example, the computer program is configured with modules including each of the functional units described above. As for the actual hardware, the CPU reads out and executes the computer program from a storage medium such as a ROM, so that each of the modules is loaded onto a main memory device and generated on the main memory device.

Furthermore, in the embodiments described above, each of the structural components of each of the illustrated devices is the functional concept and is not necessarily need to be physically configured as illustrated in the drawings. In other words, the specific forms of distribution and integration of the devices are not limited to those illustrated in the drawings, but all or some of them can be functionally or physically distributed or integrated in any unit in accordance with various kinds of load, use state, or the like. Furthermore, all or some of the processing functions performed by respective devices can be implemented by the CPU and the computer program that is analyzed and executed by the CPU, or may be implemented by hardware using wired logic.

Regarding the processing described in the above embodiments, all or several pieces of the processing described to be performed automatically can be performed manually, or all or several pieces of the processing described to be performed manually can be performed automatically using a known method. In addition to the above, the processing procedures, control procedures, specific names, and information including various kinds of data and parameters discussed in the description and drawings can be changed as appropriate, unless otherwise noted.

According to at least one of the embodiments described above, it is possible to easily and appropriately set the excitation thickness and the excitation position of the saturation pulse applied outside the imaging area within the subject in the phase encoding direction.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims.

**Claims**

1. A magnetic resonance imaging apparatus (100), comprising:

   an acquisition unit (17b) configured to acquire one-dimensional projection data in a phase encoding direction of a subject to be a target of main imaging;
   a measurement unit (17c) configured to measure a subject thickness in the phase encoding direction using the one-dimensional projection data; and
   a calculation unit (17d) configured to calculate, based on the subject thickness, an excitation thickness and an excitation position of a saturation pulse applied outside an imaging area within the subject in the phase encoding direction when performing the main imaging.

2. The magnetic resonance imaging apparatus (100) according to claim 1, wherein
   the acquisition unit (17b) is further configured to acquire the one-dimensional projection data by collecting the one-dimensional projection data in the phase encoding direction by exciting a slice imaging range including a slice of the subject captured in the main imaging.

3. The magnetic resonance imaging apparatus (100) according to claim 1 or 2, wherein
   the measurement unit (17c) is further configured to acquire a signal profile indicating a signal distribution of the subject in the phase encoding direction from the one-dimensional projection data, and measure the subject thickness outside the imaging area in the phase encoding direction based on the signal profile.

4. The magnetic resonance imaging apparatus (100) according to claim 3, wherein
   the measurement unit (17c) is further configured to specify a position where a signal of the subject becomes a prescribed value in the phase encoding direction based on the signal profile, and measure the subject thickness by measuring a distance between the specified position and an edge position of the imaging area in the phase encoding direction.

5. The magnetic resonance imaging apparatus (100) according to claim 1 or 2, wherein
   the calculation unit (17d) is further configured to calculate the excitation thickness and the excitation position of the saturation pulse based on the subject thickness, a center position of the imaging area, and a size of the imaging area.

6. The magnetic resonance imaging apparatus (100) according to claim 1 or 2, wherein
   the calculation unit (17d) is further configured to calculate a side lobe width of the saturation pulse, and calculate the excitation thickness and the excitation position of the saturation pulse such that the saturation pulse is applied with a space away from the imaging area by at least the size of the side lobe width.

7. The magnetic resonance imaging apparatus (100) according to claim 1, wherein
   the acquisition unit (17b) is further configured to acquire the one-dimensional projection data by generating the one-dimensional projection data in the phase encoding direction from imaging data for positioning the subject collected before performing the main imaging.

8. The magnetic resonance imaging apparatus (100) according to claim 1 or 2, wherein
   the one-dimensional projection data is nuclear magnetic resonance data collected by applying only a gradient magnetic field in the phase encoding direction without applying a gradient magnetic field in a frequency encoding direction and a gradient magnetic field in a slice selective excitation direction.

9. The magnetic resonance imaging apparatus (100) according to claim 1 or 2, wherein
   the saturation pulse applied outside the imaging area in the phase encoding direction is an outer volume suppression (OVS) pulse.

10. The magnetic resonance imaging apparatus (100) according to claim 1 or 2, wherein
    the excitation thickness is a thickness of the saturation pulse in the phase encoding direction.

11. The magnetic resonance imaging apparatus (100) according to claim 1 or 2, wherein
    the excitation position is a position of the saturation pulse in the phase encoding direction.

12. A magnetic resonance imaging method, comprising:

acquiring one-dimensional projection data in a phase encoding direction of a subject to be a target of main imaging (S101);

measuring a subject thickness in the phase encoding direction using the one-dimensional projection data (S102); and

calculating, based on the subject thickness, an excitation thickness and an excitation position of a saturation pulse applied outside an imaging area within the subject in the phase encoding direction when performing the main imaging (S103).

# FIG.1

EP 4 597 145 A1

# FIG.2

(A)

(B)

# FIG.3

(A) — (B) — (C)

SLICE IMAGING RANGE

PROJECTION IMAGING

1D PROJECTION DATA

PHASE ENCODING DIRECTION

IMAGING AREA

FREQUENCY ENCODING DIRECTION

SLICE SELECTIVE EXCITATION DIRECTION

OVS
IMAGING AREA
OVS

OVS
IMAGING AREA
OVS

| Pre scan | 1D Projection | CALCULATION OF OVS EXCITATION THICKNESS AND EXCITATION POSITION | MAIN IMAGING |

EP 4 597 145 A1

# FIG.4

1D PROJECTION DATA

PHASE ENCODING DIRECTION

OVS

thick_sub1

IMAGING AREA

OVS

thick_sub2

# FIG.5

IMAGING AREA

SIGNAL PROFILE

y1_zero    y1_edge

PHASE ENCODING DIRECTION

# FIG.6

PHASE ENCODING DIRECTION

OVS

X1, Y1

thick_sat1

IMAGING AREA

x, y

FOVy

OVS

X2, Y2

thick_sat2

FREQUENCY ENCODING DIRECTION

# FIG.7

START

↓

$\varsigma$S101

ACQUIRE 1D PROJECTION DATA IN
PHASE ENCODING DIRECTION

↓

$\varsigma$S102

MEASURE SUBJECT THICKNESS IN
PHASE ENCODING DIRECTION

↓

$\varsigma$S103

CALCULATE EXCITATION THICKNESS AND
EXCITATION POSITION OF OVS PULSE

↓

$\varsigma$S104

EXECUTE MAIN IMAGING USING OVS METHOD

↓

END

# FIG.8

SIDE LOBE WIDTH

RF PROFILE OF
OVS PULSE

[mm]

# FIG.9

PHASE ENCODING DIRECTION

OVS

X1, Y1

IMAGING
AREA

x, y  FOVy

OVS

X2, Y2

FREQUENCY ENCODING DIRECTION

FIG.10

Europäisches
Patentamt
European
Patent Office
Office européen
des brevets

# EUROPEAN SEARCH REPORT

**Application Number**

EP 25 15 5383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 3 995329 B2 (TOSHIBA CORP) 24 October 2007 (2007-10-24) * the whole document * | 1-12 | INV. G01R33/483 G01R33/54 |
| A | VENUGOPAL N. ET AL: "Automatic conformal prescription of very selective saturation bands for in vivo 1 H-MRSI of the prostate", NMR IN BIOMEDICINE., vol. 25, no. 4, 9 December 2011 (2011-12-09), pages 643-653, XP093280667, GB ISSN: 0952-3480, DOI: 10.1002/nbm.1780 Retrieved from the Internet: URL:https://api.wiley.com/onlinelibrary/td m/v1/articles/10.1002%2Fnbm.1780> * the whole document * | 1-12 | |

**TECHNICAL FIELDS SEARCHED (IPC)**

G01R

The present search report has been drawn up for all claims

| Place of search | Date of completion of the search | Examiner |
|---|---|---|
| Munich | 23 May 2025 | Durst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 5383

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2025

| Patent document cited in search report | | Publication date | Patent family member(s) | | Publication date |
|---|---|---|---|---|---|
| JP 3995329 | B2 | 24-10-2007 | JP | 3995329 B2 | 24-10-2007 |
| | | | JP | H11225992 A | 24-08-1999 |

For more details about this annex : see Official Journal of the European Patent Office, No. 12/82